(19) Europäisches Patentamt
European Patent Office
Office européen des brevets

(11) Publication number: **0 149 249**
**A2**

(12) # EUROPEAN PATENT APPLICATION

(21) Application number: 84116434.6

(22) Date of filing: 28.12.84

(51) Int. Cl.[4]: C 08 B 11/145
A 61 K 7/11

(30) Priority: 30.12.83 US 567388
19.10.84 US 662897

(43) Date of publication of application:
24.07.85 Bulletin 85/30

(84) Designated Contracting States:
AT BE CH DE FR GB IT LI LU NL SE

(71) Applicant: UNION CARBIDE CORPORATION
Old Ridgebury Road
Danbury Connecticut 06817(US)

(72) Inventor: Brode, George Lewis
653 Carlene Drive
Somerville 08876/USA State of New Jersey(US)

(72) Inventor: Kreeger, Russell Lowel
462 New Center Road
Somerville New Jersey 08876(US)

(72) Inventor: Merritt II, Frederick Maynard
22 Sturwood Drive
Belle Mead 08807/USA State of New Jersey(US)

(72) Inventor: Turney, Mary Elizabeth
151 South State Road
Briarcliff Manor New York(US)

(74) Representative: Weinhold, Peter, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

(54) **Polysaccharides and personal care products utilizing such polysaccharides.**

(57) Alcohol soluble and sprayable polysaccharides useful as personal care products including hair spray formulations are disclosed. The polysaccharides are cellulose ether derivatives, including those containing quaternary nitrogen, which have sufficient molar substitution and optionally cationic substitution adequate to impart alcohol solubility.

EP 0 149 249 A2

# POLYSACCHARIDES AND PERSONAL CARE PRODUCTS UTILIZING SUCH POLYSACCHARIDES

## CROSS REFERENCE TO RELATED APPLICATION

This application is a continuation-in-part of U.S. Patent Application Serial No. 567,388, filed December 30, 1983.

## BACKGROUND OF THE INVENTION

### Field Of The Invention

This invention relates to personal care products and, more particularly, to novel polysaccharides of improved alcohol solubility which may advantageously be used in a variety of personal care products.

### Description Of The Prior Art

U.S. Patent No. 3,472,840 (Stone et al.), assigned to the assignee of the present invention, discloses novel cationic quaternary-nitrogen containing cellulose ethers having a backbone of anhydroglucose units with substituent groups bearing a full positive charge spaced along this backbone. As a result of the presence of these positively charged groups imparting cationic character to the polymer, the ethers disclosed exhibit increased substantivity to a variety of substrates. Among the uses disclosed for such ethers are as flocculents, as pigment retention aids in paper-making, as antistatic fibers and fabrics, as hand stiffeners for fabrics, in cosmetic formulations, in adhesives, in printing inks and so forth.

U.S. Patent No. 3,715,428 (Quasius et al.) discloses a hair setting formulation which includes the use of the cationic cellulose ether polymers disclosed in Stone et al. The average value for the ethoxy groups in the polymers is stated to be from 1 to 2, with the average value preferred being in the range of 1.5 to 1.8. The preferred solvents for the formulations are stated to be mixtures of water with ethyl or isopropyl alcohol containing from 20 to 80% water by weight. Example 1 dissolves 0.2%, based on the weight of the solvent, of the polymeric quaternary cellulose ether salt in equal parts by weight of ethyl alcohol and water. After drying, it is stated that the hair style was found to be firmly set; and, when forcibly disarranged, the hair could readily be re-styled by combing out without appreciable flaking of the coating from the hair fibers. The Example further states that similar results were obtained by dissolving the same polymeric salt at the same concentration in water alone or in mixtures of water and ethyl alcohol varying in composition up to 90% alcohol and 10% water.

U.S. Patent No. 3,876,760 (Nersesian et al.) and U.S. Patent No. 3,959,463 (Nersesian et al.) disclose a hair dressing composition comprising a hair substantive quaternary resin and a grooming agent which is preferably incompatible with the quaternary resin. One class of the hair substantive quaternary resins disclosed are the cationic quaternary-nitrogen containing cellulose ethers disclosed in Stone et al. The composition assumes

the form of solutions of the active ingredients in an aqueous alcoholic solvent. One group of the hair fixative quaternary resins of special interest disclosed are those manufactured by the assignee of the present invention, referred to therein as JR-1 resins. These resins are described therein as 2-hydroxypropyltrimethylammonium chloride ethers of hydroxyethylcellulose. The molecular weights of the resins are stated to be in the range 200,000 to 2,000,000.

U.S. Patent No. 3,958,581 (Abegg et al.) discloses a cosmetic composition to strengthen hair which includes a cosmetic vehicle of water, ethanol, isopropanol or mixtures thereof, a cationic polymer and a divalent metal salt. Representative cationic polymers include the cationic quaternary-nitrogen containing cellulose ethers disclosed in Stone et al. Specific commercial products identified are JR-IL and JR-400.

U.S. Patent No. 3,992,336 (Faucher et al.) and U.S. Patent No. 4,018,729 (Faucher et al.) disclose articles for conditioning hair which are fabricated by blending water soluble polymers with water insoluble polymers to form interpenetrating networks so that the water soluble polymer can be extracted from the article when wet or when brought in contact with wet hair. The water soluble organic polymers disclosed include the quaternary-nitrogen containing cellulose ethers set forth in Stone et al. Specifically disclosed are the JR Resins and, more particularly, JR-125, JR-400 and JR-30M.

U.S. Patent No. 4,192,862 (Pengilly) discloses a hairspray product comprising a hair spray resin, a suitable solvent and a minor amount of a drag reducing agent. One type of polymer stated to be effective as a drag reducing agent is a very high molecular weight cationic cellulose polymer of the type described in Stone et al. Polymer JR-30M is specifically disclosed as being useful, but this is considered less useful than the other drag-reducing agents referred to (disclosed as being either ethanol soluble or methylene chloride soluble) because use requires relatively large amounts of water. Lower viscosity grades, JR-125 and JR-400, are stated to be not useful as drag reducing agents.

U.S. Patent No. 4,285,973 (Edwards) discloses a protective skin lotion in the form of an oil-in-water emulsion in which the water phase contains a quaternary ammonium cellulose ether polymer. Such polymers include the polymers disclosed in Stone et al. Specific Polymer JR products disclosed are JR-400, JR-125 and JR-30M.

A resin considered to be satisfactorily useful for a hair spray application must meet a diverse set of conditions. The resin must be capable of being sprayed in the commercial nozzle systems used for this application with the various propellants or pump systems that are being employed. Further, the resin must be alcohol soluble in the amount needed to impart the necessary performance characteristics desired, including satisfactory hair-holding properties. More

particularly, hair spray resin formulations generally require a predominantly alcohol vehicle. Only relatively minor amounts of water are typically desired so that the hair spray after application will be satisfactorily quick drying. This requires that the amount of water employed will typically be less than 10% by weight of the overall solvent (viz. - about 8% by volume), more typically no more than about 5 to 7% by weight (viz. - about 5% by volume). To impart satisfactory performance characteristics will typically require that the hair spray resin be present in an amount of at least 0.5% by weight of the formulation. Commercial hair spray formulations often include about 2% by weight of the hair spray film-forming resin and, in some cases, even higher percentages are employed.

Among the resins for hair spray applications which satisfy these requirements to some degree are the polyvinylpyrrolidone resins. However, the softness of the curls, the ease of combing and compatibility with an isobutane propellant are generally considered to be inferior by industry standards. Vinyl acetate/crotonic acid copolymers and terpolymers are used, and the properties imparted are considered to be an improvement over the polyvinylpyrrolidones.

Perhaps the state-of-the-art resin of choice is the half ethyl ester of a vinyl methyl ether/maleic anhydride copolymer, e.g.-GAF's "Gantrez ES-225". Resins of this type generally provide superior curl retention properties as well as superior propellant compatibility. However, such

resins are generally considered to impart to the hair a relatively hard feel. Moreover, and importantly, such resins flake off upon combing and impart less than an optimum sheen to the hair. Further, such resins, after chemical modification, are readily removed upon contact with commercial shampoos. Accordingly, such resins cannot impart hair conditioning properties, nor are these resins desirable for rainy weather conditions since exposure to relatively high humidity conditions or drops of water can cause tackiness (resulting in matting of the hair) which is undesirable.

It may thus be appreciated that there is substantial room for improvement in the resins used in hair spray applications. As is apparent from the widespread work in various personal care products described herein, the cationic quaternary-nitrogen containing cellulose ethers described in Stone et al. could impart considerable advantages for hair spray applications. Thus, such resins should provide a soft curl to the touch and should not flake off upon combing. Indeed, such resins should provide the ability to reset the hair; and curl retention should not be unduly diminished upon combing and resetting. The resins should not have the tackiness characteristics of the vinyl methyl ether/maleic anhydride copolymers previously described. Still further, such resins should be capable of imparting conditioning to the hair, as a relatively minor amount of the resin will be retained by the hair after use of conventional shampoos.

Despite all these potential advantages, resins of the type disclosed in Stone et al. have not been employed in hair spray applications to any extent. Resins of this type with satisfactory alcohol solubility and sprayability with conventional propellant formulations have simply not been provided. Any potential use of such resins has been restricted to either use of amounts inadequate to impart satisfactory performance characteristics or to use of amounts of water in excess of that conventionally used, with the attendant disadvantages of corrosion problems or less than satisfactory drying characteristics.

In addition, many of the same characteristics which make the cationic quaternary-nitrogen containing cellulose ethers disclosed in Stone et al. highly desirable candidates for hair spray applications apply as well to a variety of other personal care products for skin and hair conditioners, e.g. - after shaves, skin toners and the like. Indeed, hydroxyethylcellulose ethers, while not providing the substantive properties of such cationic cellulose ethers, should likewise provide desirable characteristics when employed in a variety of personal care products.

Unfortunately, many of the same problems associated with the use of such materials for hair spray applications apply as well to utilization for other personal care products. Thus, resins of this type with satisfactory alcohol solubility have not been provided. Use has accordingly been restricted

to either amounts less than desired in the particular formulation or to use with higher than desirable amounts of water.

U.S. Patent No. 3,210,251 (Klug) discloses a liquid hair preparation comprising hydroxypropylcellulose polymers and a non-toxic liquid such as a lower aliphatic saturated alcohol such as, for examples ethyl alcohol and isopropanol. These polymers are stated to be soluble in a mixture of a polar and a non-polar organic solvent, such as a mixture of a lower aliphatic alcohol and acetone. The average number of moles of reactant, i.e.- the hydroxypropyl reactant, to be combined with the cellulose per anhydroglucose unit (MS) is stated to be critical, the minimum béing at least 2.5, preferably 3-10, with 3.5-5 being specifically preferred.

U.S. Patent No. 3,839,319 (Greminger, Jr., et al.) discloses various hydroxypropyl methylcellulose ethers having a methoxyl degree of substitution of from 0.2 to 1.0 and a hydroxypropyl molar substitution of at least about 1.5 with a total combined degree of substitution of at least about 1.8. Such ethers are stated to have desirable solubility properties in anhydrous organic solvents such as methanol and ethanol. The utility disclosed for such ethers include use as thickening of paint removers, in anhydrous alcoholic solutions for coating of tablets and for coating various types of water soluble films.

U.S. Patent No. 4,084,060 (Glass, Jr. et al.) discloses a process for the synthesis of

hydroxyethylcellulose having improved resistance of enzyme catalyzed hydrolysis by employment of a controlled, optimum amount of available water for swelling the cellulose in an aqueous caustic reaction medium in hydroxyethoxylation sequences of first high and then low ratios of caustic to cellulose. The cellulose hydroxyethoxylation is continued until a molar substitution of about 3.6 to about 6.0 is reached.

Despite the desirability of utilizing hydroxyethylcellulose ethers in a variety of alcohol-based personal care products and the fact that alcohol soluble hydroxypropylcellulose ethers are available, no one has heretofore provided alcohol soluble hydroxyethylcellulose ethers. The fact that hyrdxypropylcellulose ethers which are alcohol soluble can be synthesized would not necessarily suggest that hydroxyethylcellulose ethers can be provided that are likewise alcohol soluble. The unpredictability of properties when comparing hydroxypropyl-based versus hydroxyethyl-based materials has been discussed in U.S. Patent No. 3,278,521 (Klug) (Col. 7, lines 54-63). A further indication of the differences between various properties of such materials is discussed in Klug, J. Polymer Sci.: Part C, Vol. 36, pp. 491-508, 1971, "Some Properties Of Water-Soluble Hydroxyalkyl Celluloses And Their Derivatives". For example, as shown in Figure 4, page 496, the higher equilibrium moisture content of hydroxyethylcellulose versus hydroxypropylcellulose, which is apparent at even moderate levels of

substitution, would suggest that hydroxyethylcellulose materials would not approach the low levels of equilibrium moisture content of the hydroxypropylcellulose materials even at extremely high levels of substitution. If a MS level of 2.5 as a minimum is required for hydroxypropylcellulose ethers to provide the requisite hydrophobicity to impart alcohol solubility as set forth in U.S. Patent No. 3,210,251 (Klug) and previously discussed, this data would likewise suggest that an exceedingly high MS level would be required to impart comparable hydrophobicity to hydroxyethylcellulose ethers.

<u>Objects Of The Invention</u>

It is accordingly an object of the present invention to provide polysaccharides of improved alcohol solubility and which may be utilized in a variety of personal care products, viz. - solutions, lotions and emulsions for both hair and skin care.

Another object of this invention is to provide polysaccharides which are alcohol soluble and are capable of being sprayed in conventional pressurized and pump aerosol systems.

A further object of this invention is to provide a personal care product, such as a hair spray, with acceptable curl retention which is capable of providing sheen to the hair, softness to the touch and combability following application as well as other desirable characteristics.

Other objects of this invention will be apparent from the description set forth hereinafter.

SUMMARY OF THE INVENTION

The present invention is based on the discovery that novel alcohol soluble polysaccharides may be prepared which impart highly desirable characteristics to personal care products.

These novel polysaccharides are cellulose ethers of the structural formula:

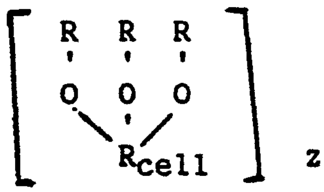

where $R_{cell}$ is the residue of an anhydroglucose unit ($C_6H_{10}O_5$), the R's may be the same or different and each R individually represents a substituent group of the formula given below, z represents the degree of polymerization and is an integer typically having a value from about 50 to about 4000. Functionally, the extent, and type of, derivatization by the R groups is selected to provide a product with the desired alcohol solubility; and the molecular weight is selected to provide the desired viscosity for a particular application.

The structural formula previously set forth represents an average so that there may be no substitution of a particular hydroxyl group, viz. - R will be H. Similarly, where, for example, methylcellulose is used, some of the R groups will be $CH_3$.

In the above structural formula, each R individually represents a substituent group of the general formula:

$$\left(CH_2-\underset{\substack{|\\R'}}{CH}-O\right)_m\left(\left(\underset{\substack{|\\R_3-\overset{+}{N}(R_2)-R_1[X^-]_{\frac{1}{V}}}}{C_x}H_{2x-1}\;O\right)_y\right)_n\left(CH_2-\underset{\substack{|\\R'}}{CH}-O\right)_p\left(C_aH_{2a}\right)_q R''$$

wherein, for the three R groups, on the average: the nitrogen atom is attached to a carbon atom not attached to another hetero atom;

<u>a</u> has a value of from 1 to 3;

<u>m</u> has a value of from 0 to 6;

<u>n</u> has an average value per anhydroglucose unit of from 0 to 3 and has a value other than 0 and adequate to impart alcohol solubility when the average value of m+p+q is inadequate to provide alcohol solubility;

<u>p</u> has a value of from 0 to 6;

<u>q</u> has a value of from 0 to 3;

the average value of m+p+q per anhydroglucose unit is one which provides the requisite alcohol solubility when n is 0;

R' is H or $CH_3$;

R" is preferably -H, but may also be a member selected from the group consisting of:

$$-\overset{O}{\overset{\|}{C}}-OH,\quad -\overset{O}{\overset{\|}{C}}-O-Na,\quad -\overset{O}{\overset{\|}{C}}-O-K \text{ and } -\overset{O}{\overset{\|}{C}}-O-NH_4$$

with the proviso that when <u>q</u> is zero, then R" is -H;

$R_1$, $R_2$ and $R_3$, taken individually, represent a member selected from the group consisting of alkyl, aryl, aralkyl, alkaryl,

cycloalkyl, alkoxyalkyl and alkoxyaryl radicals where each of $R_1$, $R_2$, and $R_3$ can contain up to ten carbon atoms, with the proviso that when said member is an alkoxyalkyl radical there are at least 2 carbon atoms separating the oxygen atom from the nitrogen atom, and with the further proviso that the total number of carbon atoms in radicals represented by $R_1$, $R_2$, and $R_3$ is from 3 to 12;

X is an anion such as chloride, bromide, iodide, sulfate, methylsulfate, sulfonate, nitrate, phosphate, acetate, etc.;

V is an integer which is equal to the valence of X;

<u>x</u> has a value of from 2 to 4;

<u>y</u> has a value of 0 to 1 with the proviso that when <u>y</u> is zero, <u>p</u> and <u>q</u> are zero and R" is H;

and with the further proviso that when R' is $CH_3$, <u>n</u> is a value other than zero.

While not illustrated in the above general formula, $R_1$, $R_2$ and $R_3$, taken together, may also represent, along with the nitrogen atom to which they are attached, a member selected from the group consisting of pyridine, α-methyl-pyridine, 3,5-dimethylpyridine, 2,4,6-trimethylpyridine, N-methyl piperidine, N-ethyl piperidine, N-methyl morpholine and N-ethyl morpholine.

In accordance with one aspect of the present invention, it has been discovered that the provision of cationic nitrogen-containing moieties substantially enhances the alcohol solubility characteristics of the resulting cellulose ethers. Further, in applications where substantivity is

desirable, this may be achieved by the selection of a value for n that will provide the substantivity needed.

Pursuant to a further aspect of the present invention, it has been discovered that novel alcohol soluble polysaccharides which are also sprayable may be provided. Functionally, the molecular weight of the polysaccharide is selected to provide a sprayable material. To this end, in the structural formula set forth, it is suitable to select a value for z within the range of from about 50 to about 1500.

## DETAILED DESCRIPTION OF THE INVENTION

### Synthetic Preparation - General Considerations

Each of the steps discussed herein may be carried out using the standard slurry techniques for preparing cellulose ethers and for quaternizing such ethers. The techniques are known; and, other than as indicated hereinafter, the particular techniques used are not considered to be of any particular importance, consistent of course with accepted synthetic practices.

### Molecular Weight Considerations

The molecular weight of the polysaccharides of this invention must be maintained within the range suitable for providing a material with the requisite alcohol solubility, and, when used for a hair spray application, for spraying with the pressurized or pump aerosol system being employed, as well. This may be determined by reference to the Brookfield viscosity (25°C.) at a 2% by weight concentration of the polysaccharide in a 95/5% (by

volume) aqueous ethanol/water solvent. The particular solvent and concentration used are somewhat arbitrary but are an approximation of the solvent and concentration typically used in a variety of personal care products, including hair spray applications, and accordingly serves as a useful reference point.

It has been found suitable to employ a polysaccharide having a viscosity of less than about 5,000 to 6,000 centipoise or so. The upper limit of useful viscosity will depend upon the performance characteristics required for the particular application, as well as the amount utilized in the formulation. Useful amounts will sometimes range up to about 5% by weight or so of the formulation; and when using amounts towards the upper part of the range, it will be generally more useful to utilize materials having viscosities somewhat less than the upper limit previously set forth.

For hair spray applications, it has been found suitable to employ a polysaccharide having a viscosity of less than about 200 centipoise or so. Sprayability increases as the viscosity is decreased. Accordingly, to enhance sprayability, the use of the novel polysaccharides of the present invention having viscosities as low as 5 to 15 centipoise or even less should enhance sprayability. On the other hand, it has been found desirable to use somewhat higher molecular weight polysaccharides to enhance the curl retention characteristics. For this reason, polysaccharides having viscosities from about 80 centipoise up to

about 150 or even 160 centipoise or so may be more desirable where enhanced curl retention characteristics are desired.

It should be appreciated that the viscosity levels set forth herein are based upon evaluations using an unoptimized nozzle system and propellant system. These novel polysaccharides exhibit to some extent pseudoplastic properties. This pseudoplastic character allows a solution of these polysaccharides in an ethanol/water solvent formulation to be sprayed at a higher viscosity than is currently considered desirable when using the state-of-the-art hair spray resin mentioned previously. In addition, aerosol systems designed to take advantage of such characteristics may increase the molecular weights that may be used somewhat. Further, when employing a pressurized aerosol system, using propellants providing increased pressures for spraying may likewise allow use of somewhat higher molecular weight materials.

As used herein, sprayability is defined as the condition wherein the dispensed hair spray formulation emits in other than a single stream. This may, of course, be readily determined.

Starting Materials

Suitable starting materials are known and are commercially available. For example, for applications where sprayability characteristics are not required, commercially available hydroxyethylcellulose materials having, at a 2 percent concentration by weight, a Brookfield viscosity (25°C.) of from about 400 centipoise or so

to about 100,000 centipoise or so may be employed. When adequately etherified as will be discussed herein, and, if desired, quaternized, the resulting polysaccharides will possess the desired alcohol solubility.

Where sprayability characteristics are desired as well, suitable starting materials comprise commercially available hydroxyethylcellulose materials having, at a 2 percent concentration by weight, a Brookfield viscosity (25°C.) of from about 5 centipoise or less to about 400 centipoise. The particular viscosity selected will usually be determined by the performance characteristics desired.

Similarly, higher molecular weight cellulose ethers which are commercially available may also be employed where sprayability characteristics are required. However, in this instance, it will be necessary to decrease the molecular weight adequately to provide the desired alcohol solubility characteristics.

Suitable techniques for degrading the molecular weights of various cellulosics are known. For example, U.S. Patent No. 2,512,338 discloses a slurry process for degrading carboxymethylcellulose by treatment with sodium hypochlorite, hypobromite or hypoiodite or hydrogen peroxide. Japanese patent 49-99780 (1974) discloses the molecular weight degradation of hydroxypropylcellulose by treatment with tertiary butyl hydroperoxide. Many other suitable techniques are known and may be employed to provide the desired viscosity when a cellulosic

material having an undesirably high molecular weight is selected as the starting material.

In addition to utilizing cellulose ethers such as hydroxyethyl and hydroxypropyl cellulose, it should be appreciated that methylcellulose, carboxymethylcellulose, ethylcellulose or their hydroxyethyl or hydroxypropyl ethers may be utilized. Many such materials are known and are commercially available.

Indeed, if desired, the starting material could be any cellulosic source such as chemical cotton, cotton linters, wood pulp, alkali cellulose, and the like. This would then require conversion to the desired cellulose ether as well as, depending upon the application involved, possibly requiring molecular weight degradation to provide the desired viscosity.

Etherification

Pursuant to the present invention, the polysaccharides must possess the requisite alcohol solubility, and, where required for a hair spray or similar application, sprayability characteristics. The former is provided by utilizing a polysaccharide ether having adequate ether groups to impart the alcohol solubility characteristic or quaternizing an inadequately alcohol soluble ether to provide the requisite alcohol solubility, as will be discussed hereinafter.

The extent of the etherification (termed herein "MS", viz. - molar substitution, and determined by the analytical technique which will be described hereinafter) should usually be sufficient

to provide, where hydroxyethylcellulose ethers are utilized, an average MS value of at least about 3.0 or so and may range up to about 5.5 or so. For most applications, it will be preferred to utilize materials having MS values of from about 3.6 to about 4.5. Thus, considering the general formula previously described and q being zero, the average value of m+p should be in the ranges set forth. Hydroxyethylcellulose etherified to this level has been found to be soluble, either alone or after quaternization in some situations, at useful concentrations in the aqueous ethanol solvents typically employed in alcohol-based personal care products such as hair spray applications and the like. Adequate solubility in other lower alkyl saturated alcohols (e.g. - methanol and isopropanol) will likely also be provided.

The degree of etherification required may be determined in a straightforward manner, viz. - simply testing the solubility at the concentration desired in the solvent system of choice. In general, the MS value needed to provide the requisite alcohol solubility will increase somewhat with the higher molecular weight polymers. On the other hand, with the lowest molecular weight hydroxyethylcellulose materials previously discussed, suitable alcohol solubility may perhaps be provided with an MS of about 3.0 or so. With hydroxyethylcellulose materials having a viscosity of about 10 centipoise or so, a MS of about 3.5 to 3.6 or so should be suitable. Materials having a viscosity of about 100 centipoise or so should

suitably have a MS of about 3.7 to 3.8 or so, while the higher molecular weight materials (viz. - a viscosity of about 5,000 to 6,000 centipoise or so) will require a MS generally of about 4.3 or more. Further, with hydroxypropylcellulose ethers or when methylcellulose, or ethylcellulose are utilized, the MS value that will be required will likely be decreased somewhat. In such instances, the particular degree of etherification and, if utilized, quaternization, needed to impart the requisite alcohol solubility may again be readily determined by simply testing the alcohol solubility of the product.

If the starting material does not have an adequate MS value to provide the requisite alcohol solubility, as is the case with commercially available materials, the requisite etherification or hydroxyalkylation may be carried out by any of the known techniques. Where hydroxyethylation is required to increase the MS value, the technique disclosed in U.S. Patent No. 4,084,060 (Glass, Jr. et al.) may suitably be employed. It is also suitable to employ any of the conventional hydroxyalkylation techniques which use higher levels of caustic: cellulose than utilized in the second stage of the Glass, Jr. et al. patent, viz. - levels as disclosed in the first stage of the Glass, Jr. et al. process may be used.

As is known, the etherified product must be neutralized to a slightly acidic pH to provide a product stable in air. Any of a variety of acids may be employed as is known. The salts which result

from the neutralization should be maintained at a minimum, as performance characteristics may be diminished somewhat as will be discussed hereinafter.

Quaternization

In accordance with one aspect of the present invention, it has been unexpectedly found, for reasons which are still unknown, that quaternization enhances the alcohol solubility of the related polysaccharide. The quaternization reaction step thus imparts to the polysaccharides of the present invention highly desirable alcohol solubility characteristics, as well as providing the necessary cationic charge to impart the requisite substantivity. Even where the unquaternized material has some degree of alcohol solubility, quaternization can significantly increase the alcohol solubility characteristics. This enhancement is reflected by an improvement in solution clarity as may be visually seen and by dimunition of the level of insoluble material. This enhancement is particularly desirable where higher molecular weight materials are utilized, relatively lower MS values are employed, or where relatively large amounts of the material are used in the particular formulation. The increase in alcohol solubility achieved by quaternization is sufficiently dramatic that, in some situations, what would be an unacceptable product can be converted to a product having eminently satisfactory alcohol solubility characteristics. The quaternization step may be effected before, subsequent to, or simultaneously with the etherification step.

In general, the level of cationic substitution (referred to herein as "CS") should be preferably maintained on the average within the range of from about 0.05 or so to about 0.6 or so, preferably in the range of from about 0.1 to about 0.4 or so. The minimum CS value will be determined by the extent of alcohol solubility enhancement required and/or by the degree of substantivity desired. The maximum level employed will generally depend upon processing and performance characteristics desired. No particular advantage has been determined for utilizing polysaccharides having excessive CS values. The cationic substitution results in the production of salts as well (e.g.,- sodium chloride when the quaternizing agent used is a chloride), so that the amount of salt resulting will be increased as the level of CS is increased. Such salts should desirably be removed to the extent considered feasible as will be discussed hereinafter. Also, from the product performance standpoint, in, for example, hair spray applications, there appears to be a tendency for performance characteristics, such as curl retention, to be improved by a decrease in the CS value. Accordingly, while polysaccharides having CS values above 0.4 may be utilized, there will generally be no reason to quaternize to this extent.

The quaternizing step may be carried out using the slurry technique disclosed in Stone et al. As is known, the diluent employed in conventional slurry preparation of cellulose ethers serves a variety of functions, including that of a

heat exchange medium and to assist in swelling the cellulosic in conjunction with the catalyst to provide a complete reaction. The diluent should likewise be basically non-reactive with the hydroxyalkylating and quaternizing agents being used.

In addition, and in accordance with this invention, the diluent employed should be one in which the polysaccharide being prepared is essentially insoluble. This is particularly critical in preparing the polysaccharides of this invention due to the relatively low molecular weights employed when used for hair spray applications and the like.

It has been found that acetone and t-butyl alcohol have the necessary characteristics to allow a facile synthesis, as compared to the isopropanol used in many of the Examples in Stone et al. The use of t-butyl alcohol is preferred for the preparation of the lower molecular weight polysaccharides of the present invention.

The quaternary ammonium compound employed to effect the cationic substitution may be any of a variety of such materials which are known. Thus, for example, 3-chloro-2-hydroxypropyltrimethylammonium chloride and the corresponding epoxide, glycidyltrimethylammonium chloride (2,3-epoxypropyltrimethylammonium chloride) are commercially available and may be employed.

It should be appreciated, however, that any quaternary ammonium compound may be employed which will impart the desired cationic charge and thus the enhanced alcohol solubility and/or substantivity. Further illustrative examples include quaternary

ammonium halides or the like wherein the straight chain or branched $C_xH_{2x-1}O_y$ moiety in the general formula previously set forth is derived from a quaternary ammonium compound having one of the following radicals: 2-chloroethyl; 3-chloropropyl; 3-chloro-2-methylpropyl; 2-chloro-1,1-dimethylethyl; 2-chloro-1-methylethyl; 4-chlorobutyl; 3-chloro-1-methylpropyl; 2-chloro-1-ethylethyl; or appropriate hydroxyl derivatives thereof. The other radicals in the quaternary ammonium compound used may be alkyl groups or any of the others previously identified. Suitable specific starting compounds of this type for the quaternization include (2-chloroethyl)trimethylammonium chloride and (3-chloropropyl)trimethylammonium chloride.

It should also be appreciated, if desired, that more than one quaternizing agent may be utilized.

Order of Reaction

When carried out, the quaternization and etherification steps may be conducted in any order, or simultaneously, in view of the similarity of reaction conditions which are effective. The particular sequence employed will, of course, dictate the particular resulting polymer structure. For example, in the general formula previously set forth, the value p will be zero if the etherification is carried out prior to the quaternization step. Similarly, if the quaternization step is carried out before the etherification, the m value will be zero for those R groups containing cationic substituents.

Product Purification

Upon neutralizing the product resulting from the etherification reaction as has been discussed, salts are produced. Likewise, as a result of the quaternization step, a salt (e.g., -sodium chloride) is formed, also contaminating the resulting product.

It has been found desirable to purify the resulting cationic polysaccharide to minimize the content of such salts. Repeated washings with a solvent such as acetone/water may be utilized (5-25 percent water by weight being exemplary). It is thus desirable to maintain the salt content, measured as percent ash, below about 10 percent by weight or so. More preferably, the salt content should be maintained no more than about 3 to 7 percent or so. The lower salt-containing materials thus appear to be less sensitive to humidity.

In the wash or extraction step and further drying to provide the finished product, some of the solvent (probably mostly water) may be retained in minor amounts within the polymer. This may be tolerated to some extent; it is, however, desirable to maintain the entrapped solvent content (measured as percent volatiles) below about 8 percent or so, more preferably below about 5 percent or so, to minimize handling problems. To further facilitate handling of the dried, solid product, it may be useful to add a flow aid, such as, for example, silica, as is known.

Further Synthetic Steps

As can be seen from the general formula previously set forth for the novel polysaccharides

of the present invention, and considering a specific reaction site at which etherification and quaternization have been effected, q will have a value of zero and R" will be hydrogen without synthetic steps being carried out beyond what have been previously described. However, if desired, the resulting product could be capped, for example, by using conventional techniques with methyl chloride or the like. Likewise, the R" group can be chemically modified, if desired, by techniques known in the art. Such further steps may be employed, for example, to alter somewhat the polymer rheology.

Alcohol Solubility

The extent of alcohol solubility will, of course, vary considerably depending upon a variety of parameters including, for example, the molecular weight of the polysaccharide, the type and level of substitution on the polysaccharide, and the relative amount of water, if any, included with the ethanol (or other organic liquid) to form the solvent carrier for the particular application involved. Solution clarity is certainly an indication of the adequacy of solubility, and the preferred materials of this invention appear visually to be essentially clear.

Further, as has been alluded to herein, upon being dissolved in the solvent employed, some amount of insoluble material (likely polymeric in nature) will result. While perhaps filtration, centrifuging, or the like can reduce the level somewhat, it is, of course, preferred to provide materials with as low a level of insolubles as

possible, for aesthetic and other considerations. Likewise, the extent of insolubles is a prime indication of the degree of alcohol solubility.

As used herein, alcohol solubility is defined by dissolving 2% by weight of the polysaccharide in a 95/5% by volume ethanol/water solvent. The polysaccharide is considered to be alcohol soluble if the level of insolubles is below about 10% by weight of the polysaccharide dispersed in the solvent. The most preferred polysaccharides of the present invention exhibit alcohol solubility characteristics such that the level of insolubles is less than about 1% by weight. It is certainly preferred to provide materials having a level of insolubles less than about 2% by weight. Some applications will tolerate insoluble levels of up to 5% by weight, and a level of insolubles of 10% by weight is considered about the practical useful limit.

The level of alcohol solubility exhibited by the polysaccharides of the present invention is more than adequate to provide highly desirable characteristics when employed in the alcohol/water solvent-based systems used in a wide variety of personal care products, as will be more fully discussed hereinafter. As may be appreciated, even when the alcohol solubility in accordance with the procedure described hereinbefore results in an undesirably high level of insolubles, use in personal care product formulations can provide desirable products essentially free of insolubles, due to higher water content in the solvent system

utilized and/or due to the polysaccharide being incorporated in an amount lower than that used in the test procedure described.

Formulation

The personal care product formulations of the present invention comprise the novel alcohol soluble polysaccharides of the present invention, an alcoholic solvent (which may contain up to about 50% by weight of water or so based upon the total weight of the solvent) for the polysaccharides, one or more active ingredients depending upon the personal care product involved, and, optionally, a variety of auxiliary agents employed in such formulations. Thus, a variety of personal care products (i.e. - skin and hair conditioners), such as, for example, after-shave lotions, skin toners, and the like employ formulations which utilize alcohol-based carriers.

The ability to provide polysaccharides that are soluble in such carriers allows such polysaccharides to be incorporated into these formulations to impart the highly desirable skin and hair conditioning characteristics attributed to polysaccharides of this type. Functionally, the amount of the polysaccharide utilized should be adequate to impart the conditioning or other characteristics desired for the particular application. In some formulations, this amount may be as low as about 0.05% by weight of the formulation, while other formulations may employ from about 1% to about 5% by weight.

The composition of the solvent system used will vary depending upon the requirements of the particular application. Some personal care products use an alcohol solvent system which may contain up to about 50% by weight water. More commonly, such solvent systems include up to about 30% by weight water or so. Also, some personal care products, e.g. - hair sprays, can generally tolerate less than about 10% by weight water, due to the desire to provide relatively quick drying characteristics.

Still further, a variety of personal care products are in the form of emulsions which employ alcohol-based carriers. The polysaccharides of the present invention may likewise be utilized in such products and their alcohol solubility characteristics may increase the amount of the polymer which can be incorporated. Indeed, the polysaccharides of the present invention may be utilized in any alcohol-based personal care product where the conditioning and other characteristics of such polysaccharides are desired.

When used as a hair spray, the hair spray formulation of the present invention comprises the novel alcohol soluble, sprayable polysaccharides of this invention, an alcoholic solvent for the polysaccharides and, when a pressurized aerosol system is utilized, a propellant. The amounts of these and other auxiliary components may be varied as desired depending upon the product performance characteristics desired.

The minimum amount of the polysaccharide employed will be dependent upon the amount required to achieve the particular performance

characteristics considered important for the particular product. Where curl retention characteristics and other aesthetics are considered important, the novel polysaccharides will normally be employed in an amount of at least about 0.5% by weight of the formulation, more typically about 1% by weight (excluding the impurities previously discussed). The maximum amount will generally be determined by sprayability and/or the particular sprayability characteristics desired. The polysaccharide level may accordingly be up to about 3% by weight or so, perhaps up to about 5% by weight.

The solvent employed, as has been discussed herein, will typically be an aqueous alcohol solution, ethanol generally being the solvent of choice. However, other alcohols may, of course, be employed, if desired, so long as the polysaccharide is soluble therein at the amount required.

The amount of water included, if any, will generally be less than about 10% by weight of the total solvent, more typically in the range of from about 5 to about 7% by weight or so. In general, the amount of water employed will be primarily dictated by corrosion considerations as well as the drying characteristics desired upon application to the hair.

When using a pressurized aerosol system, any of the conventional propellants useful for aerosol applications may be employed. The particular propellant composition may be widely varied, as desired. In conventional hair spray applications at present, where environmental

conditions dictate, liquified gaseous isobutane is the typical material of choice. The amount of isobutane utilized will generally be in amounts of 30% or less by weight of the total composition; however, the amount employed may be varied as desired to provide the sprayability considered desirable.

As is known, the amount of liquified propellant employed should be within the level of compatibility of the system for the particular propellant or propellants used. This may be visually determined for a particular system. More specifically, the tolerable maximum propellant level may be determined by adding propellant until the composition turns cloudy. Above this level, clogging of the nozzle system may result. Reducing a potential clogging problem may also perhaps be achieved by high speed centrifuging, filtration or the like.

Where environmental restrictions allow, other conventional liquified, halogenated hydrocarbon gaseous propellants may be used. Suitable examples include trichloromonofluoromethane, dichlorodifluoromethane, tetrachlorodifluoroethane, trichlorotrifluorethane, dichlorotetrafluoroethane, and the like.

If desired, gaseous propellants, as are known, may be employed as the propellant or as a part of the total propellant, more typically the latter. As an illustrative example, propane may be suitably employed. Further, nitrogen, nitrous oxide or carbon dioxide may likewise be used.

Indeed, the particular propellant composition may be tailored to the particular hair spray formulation. For example, utilizing a propellant composition which will provide higher dispensing pressures than the pressures achieved with isobutane may be more desirable for the relatively higher viscosity compositions to enhance sprayability.

In any of the formulations for skin or hair care of this invention, a variety of auxiliary components may be utilized, as is known. By way of illustration, a fragrance (perfume) is often employed. Other cosmetic additives such as humectants (moisturizers), emollients, conditioning agents, and the like may also be included. Still further, it may be desirable to include a charge neutralizing agent.

Product Performance Characteristics

The alcohol soluble polysaccharides of the present invention may accordingly be incorporated into a variety of personal care products to impart their known and highly desirable skin and hair conditioning attributes. Thus, as is known, such polysaccharides function to condition the skin by imparting desirable aesthetics as well as providing the ability to ameliorate the action of numerous chemical irritants and imparting a desirable, subtle feel to the skin. Such polysaccharides also function to condition hair, as by providing, for example, desirable feel and appearance, mending split ends and the like.

The hair care formulations of the present invention retain the many known desirable attributes of polysaccharides for personal care applications. Thus, after application, a highly desirable sheen is imparted to the hair which is extremely soft to the touch, particularly in comparison to certain of the other conventionally used hair care resins. The hair is readily combable without flaking and exhibits a lack of tackiness when wet, as would result when one is caught in the rain or the like.

Further, where the cationic polysaccharides are employed, the hair care formulation will afford conditioning and the ability to reset the curl as well. There is thus the ability of the polysaccharide film to remain with the hair, even after washing, in amounts which will provide conditioning without causing undesirable buildup.

Still further, the hair care formulations of this invention impart what are considered to be acceptable curl retention characteristics. Humidity effects will reduce the curl retention characteristics somewhat, but the effects may be lessened by utilizing the relatively higher molecular weight polysaccharides as the resin of choice.

The following two sections (viz. - POLYMER CHARACTERIZATION PARAMETERS and PERFORMANCE TESTING PROCEDURES) describe and define the various parameters of the polysaccharides of this invention, many of which have previously been discussed and will be exemplified in the following Examples, and define the performance tests utilized in evaluating

the polysaccharides:

POLYMER CHARACTERIZATION PARAMETERS

MS - Molar Substitution: MS indicates the extent of etherification undergone by a cellulose polymer. Etherification introduces ether linkages into the chains branching off the cellulose backbone. MS measures the average mole fraction of ether linkages present per anhydroglucose unit (i.e. - the polymer repeat unit). MS is determined analytically by the well known Zeisel-Morgan method used for hydroxyethylcellulose materials.

%N - % Nitrogen indicates the average weight per cent of nitrogen added per repeat unit (viz. - anhydroglucose unit) in the quaternized polymer product. %N measures the extent of quaternization and, therefore, the extent of cationization in the end product. %N is determined analytically by the standard Dohrmann or Kjeldahl methods. In the ensuing Examples, the particular method used will not be identified as the results are considered to be interchangeable. %N is reported relative to a pure end product and so reflects a correction for the impurities actually contained in the product tested.

CS - Cationic substitution: CS indicates the extent to which a cellulose polymer is quaternized. CS measures the average mole fraction of quaternary nitrogens per polymer repeat unit. When hydroxyethylcellulose (HEC) is the cellulosic starting material, CS is calculated from the following relationship:

$$CS = \frac{Wt_N \cdot MW_{HEC}}{AW_N + Wt_N(MW_{HEC} - MW_{QHEC})}$$

where $MW_{HEC}$ = molecular weight of the HEC, $MW_{QHEC}$ = molecular weight of the quaternized HEC; $AW_N$ = 14 = the atomic weight of nitrogen, and $Wt_N$ is the weight fraction of nitrogen determined by the Dohrmann or Kjeldahl methods.

$\eta$ - Viscosity: Cellulose derivatives are characterized using viscosities reported in centipoise (cps). Viscosities are measured using Cannon-Fenske or Brookfield LVT viscometers. Solutions are made to 2% by weight of pure polymer (i.e., corrected for impurities) in an aqueous ethanol solution. The viscosities for Examples 1 to 5 presented in Table IA reflect correction for ash and volatile impurities and were measured in 95% by volume aqueous ethanol solutions. The viscosities for Examples 6 to 14 presented in Tables IIA and IIIA reflect correction for ash, volatile and other impurities insoluble in aqueous ethanol; these viscosities were measured in 95/5 (V/V) aqueous ethanol solutions.

Viscosities can be measured with either Cannon-Fenske (Examples 1 to 5) or Brookfield LVT (Examples 6 to 14) Viscometers. Viscometer variables such as the bore of the Cannon-Fenske capillary and the spindle and velocity (spindle speed in revolutions per minute) for the Brookfield viscometer were selected to allow measurement in reasonable times or acceptable percent of scale readings, respectively, as is standard analytical practice.

% Ash - % ash is the percent by weight of ash (i.e. - the salts formed during the synthesis) present and is measured relative to dry polymer.

% Volatiles - % volatiles is the weight percent of volatile materials trapped in the polymer end product.

% Insolubles - % Insolubles is the weight % of all other impurities insoluble in a 95/5 (V/V) (93.76/6.24 wt./wt.) ethanol/water solution.

Methods and procedures for determining MS, n, % ash, and % volatiles are described in the 1981 book of ASTM Standards, Part 21 Cellulose, Leather, Flexible Barrier Materials - Standard Methods of Testing Hydroxyethylcellulose, Designation D2364-75 (reapproved 1979), page 274.

## PERFORMANCE TESTING PROCEDURES

### Hair Spray Application

Three tests are routinely conducted to evaluate the efficacy of a resin for a hair spray application. The tests measure the ability of the resin to keep hair curled, its compatibility with isobutane and its ability to spray from a pressurized aerosol system. The tests, nominally designated as % Curl Retention, Isobutane Compatibility and Sprayability, are described as follows:

### % Curl Retention

This test measures the ability of a hair spray resin to hold a hair sample in a curl after the resin is applied and the curl rolled. The resin is applied manually and not by spray to facilitate a more uniform application of the resin to the hair.

The test is conducted under constant temperature and humidity conditions and measures the loss of curl over time.

The test procedure and determination of % curl retention are as follows:

Ten inch tresses of virgin brown hair are washed with 2 grams of shampoo (15% by weight aqueous triethanolamine lauryl sulfate) and dried in a commercial hair dryer for one hour. The tresses are cut to 22 cm. in length and combed through. One ml. of sample resin solution is applied to the tress manually and it is again combed through. A commercial end paper is applied and the tress is wound around and clipped to 2x7 cm. roller. The rolled tress is dried for 30 minutes and then brought to the constant temperature and humidity test conditions over 15 minutes. The tress is slid gently from the roller, the end paper is removed and the horizontal length of the curl measured.

The test is begun by hanging the curl in air and continues by monitoring the change in the length over time. Measurements are taken at 15 minute intervals for one hour. Data is recorded as percent curl retention. % curl retention is calculated from the following relationship:

$$\% \text{ Curl Retention} = \frac{L_I - L_T}{L_I - L_C} \times 100\%$$

wherein: $L_I$ = Initial length of the tress;
$L_C$ = Initial length o the curl;
$L_T$ = Length of curl at specified time.

% Isobutane Compatibility

This test measures the solubility of isobutane, a propellant normally used in pressurized aerosol sprays, in an aqueous alcohol solution of hair spray resin. Data is recorded as the weight % isobutane that will dissolve in a 1% by weight solution of resin dissolved in aqueous ethanol. The data presented in Table IB is for samples made to a 1% pure resin correcting for ash and volatile impurities; data presented in Tables IIB and IIIB is corrected for ash, volatiles and other impurities insoluble in a 95/5 (V/V) ethanol/water solution.

The test procedure is as follows:

A solution of 1% by weight sample resin dissolved in aqueous ethanol is prepared. Gels and impurities are removed by centrifugation. 35 grams of solution are weighed into a standard compatibility tube which is then sealed, and a valve is inserted. Isobutane propellant is titrated into the tube until the appearance of a persistent haze. The weight of the propellant added is determined, and the weight percent calculated.

Sprayability

This test measures the ability of an aqueous ethanol solution of hair spray resin and propellant to spray from an aerosol container. Sample mists are compared subjectively to commercial products considered to have superior spray properties. Samples prepared for the Isobutane Compatibility test are sprayed at alcohol sensitive paper from a standard distance for a fixed time. The spray pattern, drying rate and droplet formation

characteristics are assessed individually before a general evaluation is made. Sprays are judged to be poor, marginal, good or excellent in comparison to commercial standards.

The commercial hair spray used as a control was GAF's "Gantrez ES-225" resin a 2% by weight solution of in an aqueous ethanol solvent.

Alcohol-Based Hair Conditioner Application

Six tests are routinely conducted to evaluate the performance of a resin as a hair conditioner. Subjective measurements of feel and appearance (wet and dry) and quantitative measurements of wet and dry combability and fly away are compared on hair tresses treated with alcoholic/resin solutions and with alcohol solutions only.

Preparation Of Hair Tresses

A 1 gram, 10-inch swatch of commercially available virgin brown hair is treated with 1 gram of a 1% polymer solution in a 95/5% by volume ethanol/water solvent. The swatch is attached to a board while still wet and is tested for wet combability, wet feel, and wet appearance. After drying with a hair dryer, the tress is tested for dry combability, dry appearance and fly away after equilibrating at about 70°F. and 60% R.H.

Control hair tresses are prepared and tested in a like manner, employing the same solvent with no resin.

Combability

A comb is passed through either a wet or dry hair tress at a 90° angle, and the distance the

comb travels between the point of attachment and any snarl is measured in inches.

Wet Feel

Wet feel is rated subjectively versus a control on a 1-5 scale. Ratings are as follows: 1- tacky, greasy, or slimy; 2- no slip and/or greasy; 3- slightly slippery; 4- moderately soft, silky and/or slightly slippery; 5- soft, silky, and slippery. The best performance is indicated by a 5 rating.

Appearance

Wet and dry appearance are rated subjectively versus the controls on a 1-5 scale with best performance indicated by a 5 rating. Ratings are as follows: 1- dull; 2- slight sheen; 3- good sheen; 4- very good sheen; 5- excellent sheen.

Fly Away

Fly away is a measure of electrostatic charge build-up on dry hair after combing. A dry hair tress is rapidly combed 10 times at a 90° angle; and the extent of spread is measured in two ways: (1) the main bundle width in inches is measured, and (2) the distance from the outer bundle edge to the most extreme strand is measured in inches. The total of the two numbers represents the rating with best performance being indicated by the lowest number.

Examples 1-5

Examples 1 to 5 were prepared by ethoxylation of low molecular weight hydroxyethylcellulose (HEC), purifying the product and further reacting it with

3-chloro-2-hydroxypropyltrimethylammonium chloride (CHPTA). All examples were similarly prepared except that the amounts of reagents were varied to effect changes in MS, CS and product viscosities.

The starting material for Examples 1 to 5 was a commercially available hydroxyethylcellulose characterized by a 5% solution viscosity in water of from 113 to 150 cps. MS, CS and solution viscosities for the products range from 2.8 to 4.0, 0.16 to 0.61 and 6.5 to 13.6 cps respectively. Synthesis and analytical data is presented in Table IA, with performance data presented in Table IB.

As can be seen from Tables IA and B, the data supports a tendency for the curl retention capability to be better for low rather than high MS (c.f., Examples 1 and 4) and for low rather than high CS (c.f., Examples 1 and 2).

TABLE IA

EXAMPLES 1-5

| | | | | | | | | Ethoxylation Conditions (1) | | Quaternization Conditions (1) | |
|---|---|---|---|---|---|---|---|---|---|---|---|
| Ex. | MS | % N | CS | (4) 2% Visc. | % Ash | % Vol. | % Insol. | NaOH:HEC (2) | EO:HEC | NaOH:HEC | CHPTA (3) :HEC |
| 1 | 2.8 | 0.84 | 0.19 | 13.6 | 7.17 | 1.01 | -- | 1.4 | 1.6 | 0.7 | 0.3 |
| 2 | 2.8 | 2.11 | 0.56 | 8.1 | 12.44 | 0.63 | -- | 1.4 | 1.6 | 1.0 | 0.75 |
| 3 | 3.4 | 1.29 | 0.33 | 9.4 | 10.97 | 0.75 | -- | 1.4 | 2.65 | 1.0 | 0.50 |
| 4 | 3.9 | 0.61 | 0.16 | 9.0 | 9.53 | 0.88 | -- | 1.4 | 3.53 | 0.7 | 0.30 |
| 5 | 4.0 | 1.99 | 0.61 | 6.5 | 11.61 | 0.13 | -- | 1.4 | 4.16 | 1.0 | 0.80 |

(1) mole ratio based on contained HEC.

(2) Examples 1 to 5 were prepared from a commercially available HEC characterized by a 5% aqueous solution viscosity of from 113 to 150 cps.

(3) 3-chloro-2-hydroxypropyltrimethylammonium chloride.

(4) Viscosities were measured in 95% by weight aqueous ethanol solution with a Cannon-Fenske viscometer for ash and volatile impurities. Samples were prepared as 2% by weight pure resin correcting.

TABLE IB

EXAMPLES 1-5

| | PERFORMANCE TESTS | | | | | |
|---|---|---|---|---|---|---|
| | % Curl Retention(1) at | | | | % Isobutane Compatibility | Sprayability |
| Example | 15' | 30' | 45' | 60' | | |
| 1[a] | 82 | 76 | 74 | 68 | 16 | Marginal to good |
| 2[a] | 67 | 49 | 45 | 41 | 17 | Marginal to good |
| 3[b] | 84 | 77 | 74 | 70 | 20 | Marginal to good |
| 4[a] | 64 | 53 | 44 | 40 | 23 | Marginal to good |
| 5[a] | 55 | 44 | 41 | 31 | 23 | Marginal to good |
| ES-225[2,a] | 88 | 83 | 80 | 77 | 25 | Excellent |
| ES-225[2,b] | 83 | 74 | 73 | 69 | 25 | Excellent |

[a]Measurements made at 68°F., 65% relative humidity.

[b]Measurements made at 70°F., 63% relative humidity.

(1) As 1% solutions.

(2) Gantrez ES-225 control at 2% solution concentration.

## Examples 6-10

Examples 6 to 10 were prepared by ethoxylation and purification of a high molecular weight HEC. Ethoxylation was followed by degradation to lower the molecular weight and quaternization with CHPTA. All Examples were similarly prepared except that the amounts of reagents were varied to effect changes in MS and product solution viscosities.

The starting material for Examples 6 to 10 was a commercially available HEC characterized by a 1% water solution viscosity of from 2400-3000 cps. MS and product solution viscosities range from 3.4 to 4.3 and 17 to 197 cps respectively. Synthesis and analytical data for Examples 6 to 10 is presented in Table IIA, with performance data presented in Table IIB.

As can be seen from Tables IIA and B; the data tends to support the conclusion that curl retention is better for resins with low MS (c.f., Examples 6 and 9). The data also tends to show that curl retention and sprayability vary conversely with solution viscosity. Also, curl retention apparently improves at high rather than low viscosity but sprayability appears better at low viscosity (c.f., Examples 6 and 7).

TABLE IIA

EXAMPLES 6-10

| Ex. | MS | % N | CS | [4] 2% Visc. | % Insol. | % Ash | % Vol. | Ethoxylation Conditions[1]: NaOH:HEC[2] | Ethoxylation Conditions[1]: EO:HEC | Quaternization Conditions[1]: NaOH:HEC | Quaternization Conditions[1]: CHPTA[3]:HEC |
|---|---|---|---|---|---|---|---|---|---|---|---|
| 6 | 4.3 | 1.09 | 0.31 | 197 | nil | 2.26 | 1.14 | .283 | 2.7 | 1.07 | .647 |
| 7 | 4.3 | 1.46 | 0.43 | 21 | .24 | 6.58 | 1.86 | .283 | 2.7 | 1.07 | .647 |
| 8 | 3.9 | 1.30 | 0.36 | 93 | .05 | 3.60 | 0.30 | .283 | 2.2 | .96 | .576 |
| 9 | 3.4 | 1.31 | 0.34 | 161 | .59 | 3.25 | 1.73 | .283 | 2.0 | .96 | .576 |
| 10 | 3.4 | 1.32 | 0.34 | 17 | .47 | 2.82 | 9.22 | .283 | 2.0 | .90 | .542 |

(1) Mole ratios based on contained HEC.

(2) Examples 6 to 10 were prepared from a commercially available hydroxyethyl cellulose (HEC) characterized by a 1% aqueous solutioin viscosity of from 2400 to 3000 cps.

(3) 3-chloro-2-hydroxypropyltrimethylammonium chloride.

(4) Viscosities were measured in 95/5 (V/V) ethanol/water solutions with a Brookfield LVT viscometer. Samples were prepared as 2% by weight pure resin correcting for ash, volatile and impurities insoluble in a 95/5 (V/V) aqueous ethanol solution.

TABLE IIB

EXAMPLES 6-10

| Example | PERFORMANCE TESTS | | | | | |
|---|---|---|---|---|---|---|
| | % Curl Retention[(1)] at | | | | % Isobutane Compatibility | Sprayability |
| | 15' | 30' | 45' | 60' | | |
| 6 | 54 | 45 | 40 | 36 | 19 | Poor |
| 7 | 32 | 26 | 18 | 10 | 24 | Good |
| 8 | 65 | 60 | 58 | 50 | 20 | Marginal |
| 9 | 84 | 80 | 78 | 74 | 19 | Poor |
| 10 | 68 | 55 | 49 | 40 | 18 | Good |
| [(2)]ES-225 | 88 | 85 | 84 | 84 | 25 | Excellent |

(1) As 1% solutions at 72°F. and 60% relative humidity.

(2) Gantrez ES-225 Control at 2% solution concentration.

Examples 11 to 14

Examples 11 to 14 were prepared by ethoxylation of two commercially available high molecular weight HEC materials. Examples 11 and 12 used an HEC characterized by a 1% water solution viscosity of from 2400-3000 cps. Examples 13 and 14 used an HEC characterized by a 2% water solution viscosity of from 4800-6000 cps. In all four Examples, ethoxylation was followed by degradation to reduce the molecular weights and quaternization with CHPTA. All Examples were otherwise similarly prepared except the amounts of reagents were varied to effect changes in CS and product solution viscosities. Synthesis and analytical data for Examples 11 to 14 is presented in Table IIIA, with performance data presented in Table IIIB.

As can be seen from Tables IIIA and B, the data tends to support the conclusion that curl retention characteristics improve when CS and solution viscosity are coupled so that CS is kept low while viscosity is kept high. As seen in Table IIIB, the composition of Example 14 was not sprayable. While this was the result obtained, this is considered to be an inappropriate result, as a hair spray formulation using the polysaccharide of Example 14 should be sprayable in view of the other experimental work carried out. This Example is accordingly included simply for the sake of completeness.

TABLE IIIA

EXAMPLES 11-14

| | | | | | | | | ETHOXYLATION CONDITIONS (1) | | QUATERNIZATION CONDITIONS (1) | |
|---|---|---|---|---|---|---|---|---|---|---|---|
| Ex. | MS | % N | CS | (4) 2% Visc. | % Ash | % Vol. | % Insol. | NaOH:HEC (2) | EO:HEC | NaOH:HEC | CHPTA (3): HEC |
| 11 | 3.62 | 1.14 | 0.30 | 59 | 3.45 | 2.19 | .83 | .283 | 1.46 | 1.00 | .60 |
| 12 | 3.79 | 0.54 | 0.13 | 168 | 3.00 | 5.78 | .55 | .283 | 1.44 | 1.00 | .29 |
| 13 | 3.85 | 1.17 | 0.32 | 38 | 5.17 | 1.99 | .04 | 1.417 | 3.33 | 1.00 | .67 |
| 14 | 3.77 | 0.46 | 0.11 | 99 | 4.18 | 1.61 | .93 | 1.417 | 3.33 | 1.00 | .29 |

(1) Mole ratios based on contained HEC
(2) Examples 11 and 12 were prepared from a commercially available HEC characterized by a 1% water solution viscosity of from 2400 to 3000 cps. Examples 13 and 14 were prepared from a commercially available HEC with a 2% water solution viscosity of from 4800 to 6000 cps. All samples were prepared as 2% by weight pure resin correcting for ash, volatile and impurities insoluable in a 95/5 (V/V) aqueous ethanol solution.
(3) 3-chloro-2-hydroxypropyltrimethylammonium chloride
(4) Viscosities were measured in 95/5 (V/V) ethanol/water solutions

## TABLE IIIB

## EXAMPLES 11-14

| | PERFORMANCE TESTS | | | | | |
|---|---|---|---|---|---|---|
| | % Curl Retention[(1)] at | | | | % Isobutane Compati-bility | Spray-ability |
| Example | 15' | 30' | 45' | 60' | | |
| 11[a] | 83 | 76 | 71 | 68 | 21 | Poor |
| 12[b] | 92 | 90 | 84 | 81 | 20 | Good |
| 13[c] | 89 | 87 | 85 | 85 | 18 | Poor |
| 14[d] | 92 | 90 | 90 | 88 | 18 | Streams |
| [(2)]ES-225 | 90 | 89 | 89 | 89 | 25 | Excellent |

[a]Measurements were made at 72°F. and 59% relative humidity.

[b]Measurements were made at 70°F. and 62% relative humidity.

[c]Measurements were made at 70°F. and 50% relative humidity.

[d]Measurements were made at 70°F. and 55% relative humidity.

[(1)]As 1% solutions

[(2)]Gantrez ES-225 control at 2% solution concentration

Examples 15-25

These Examples illustrate the enhancement of alcohol solubility achieved through quaternization of hydroxyethylcellulose materials, as well as the effects of molecular weight.

Examples 15-23 employed the procedure described in Examples 11-14, while Examples 24 and 25 utilized the procedure set forth in Examples 1-5. Table IV sets forth the synthetic and analytical data:

Table IV

| Ex. | MS | CS | Visc. - 2% | % Insol. |
|---|---|---|---|---|
| 15 | 3.53 | 0 | 135 | 15.96 |
| 16 | 3.57 | 0.14 | 87 | 2.38 |
| 17 | 3.59 | 0 | 17 | 0.24 |
| 18 | 3.56(1) | 0.12 | 18 | 0 |
| 19 | 3.74 | 0 | 205 | 1.36 |
| 20 | 3.79 | 0.13 | 168 | 0.55 |
| 21 | 3.75 | 0 | 230 | 20.34 |
| 22 | 3.75 | 0 | 21 | 1.66 |
| 23 | 3.74 | 0.13 | 166 | 0.95 |
| 24 | 3.80 | 0 | 15 | 17.80 |
| 25 | 3.80 | 0.15(2) | 13 | 1.99 |

(1) Average of 3 values since analytical determination was considered to be in error.
(2) Calculated value, based upon an average reaction efficiency obtained from similar preparations.

As can be seen from Table IV, enhancement in alcohol solubility is provided by quaternization, as indicated by the reduced levels of insolubles.

## Examples 26-37

These Examples show the alcohol solubility enhancement obtained due to quaternization of higher molecular weight hydroxyethylcellulose materials and, as well, hydroxypropylcellulose materials.

Examples 26-33 utilized the preparation set forth in Examples 1-5; Examples 34-37 describe the hydroxypropylation of cotton linters, with or without quaternization, also utilizing the procedure of Examples 1-5. Table V sets forth the synthetic and analytical data:

Table V

| Ex. | MS(1) | CS | Visc. - 2% | % Insol. ETOH/$H_2O$ | ETOH |
|---|---|---|---|---|---|
| 26 | 4.1 | 0 | 1,437 | 1.81 | 20.42 |
| 27 | 4.1 | 0.22 | 800 | 0.09 | 9.00 |
| 28 | 4.8 | 0 | 1,157 | 0.57 | 6.96 |
| 29 | 4.8 | 0.21 | 709 | 0.14 | 4.87 |
| 30 | 3.8 | 0 | 6,460 | 37.09 | -- |
| 31 | 3.8 | 0.18 | 4,653 | 7.25 | -- |
| 32 | 4.1 | 0 | 5,813 | 18.54 | -- |
| 33 | 4.1 | 0.16 | 4,467 | 1.30 | -- |
| 34 | 1.6 | 0 | 37,300(2) | 82.67(3) | -- |
| 35 | 1.6 | 0.18 | 38,875(2) | 11.43(3) | -- |
| 36 | 3.0 | 0 | 128 | 21.23 | -- |
| 37 | 3.0 | 0.07 | -- | 5.66 | -- |

(1) Values are based upon material balances.

(2) Determined in water.

(3) Determined in 60/40 wt./wt. ethanol/water rather than the standard low water-ethanol solvent used for the other Examples and previously described.

As seen from Table V, the quaternization of the materials resulted in enhancement of alcohol solubility, with the extent of enhancement increasing with the viscosity of the material.

Examples 38-47

Performance as a hair conditioner delivered from a 95/5% by volume ethanol/water solution was done on five hair tresses treated with a polysaccharide of the present invention having the following characteristics: a MS of approximately 4.3, a CS of approximately 0.4, and a 2% solution viscosity in 95/5% by volume ethanol/water of approximately 310 centipoise -- prepared as in Examples 11-14 (Examples 38-42). As a control, five hair tresses were treated with only the 95/5% by volume ethano/water solvent (Examples 43-47).

The results are shown in Table VI:

TABLE VI

| Ex. | Wet Comba-bility | Wet Feel | Wet Appear-ance | Dry Comba-bility | Dry Appear-ance | Fly Away* |
|---|---|---|---|---|---|---|
| 38 | 10 | 5 | 5 | 10 | 5 | 1 + 1.5 = 2.5 |
| 39 | 10 | 5 | 5 | 10 | 5 | 2 + 1.5 = 3.5 |
| 40 | 10 | 5 | 5 | 10 | 5 | 1 + 1.5 = 2.5 |
| 41 | 10 | 5 | 5 | 10 | 5 | 1 + 0.5 = 1.5 |
| 42 | 10 | 5 | 5 | 10 | 5 | 3 + 0.5 = 3.5 |
| Avg. | 10 | 5 | 5 | 10 | 5 | 2.7 |
| 43 | 5.5 | 2 | 2 | 6.0 | 1 | 3 + 1.0 = 4.0 |
| 44 | 6.0 | 2 | 2 | 5.5 | 1 | 3 + 1.5 = 4.5 |
| 45 | 5.5 | 2 | 2 | 8.0 | 1 | 3 + 0.5 = 3.5 |
| 46 | 5.0 | 2 | 2 | 8.0 | 1 | 2 + 1.0 = 3.0 |
| 47 | 4.0 | 2 | 2 | 7.0 | 1 | 3 + 0.5 = 3.5 |
| Avg. | 5.2 | 2 | 2 | 6.9 | 1 | 3.7 |

* The first number is a measurement of a bundle in inches and the second number is a measurement of the bundle to the extreme, also in inches.

As can be seen, the formulation utilizing the polysaccharides of the present invention, provided, in relation to the control, superior performance.

**WE CLAIM:**

1. A polysaccharide of the structural formula:

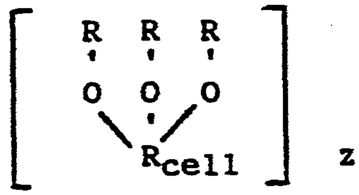

wherein:

$R_{cell}$ is the residue of an anhydroglucose unit;

z is an integer having a value of from about 50 to 4000;

each R individually represents a substituent group of the general formula:

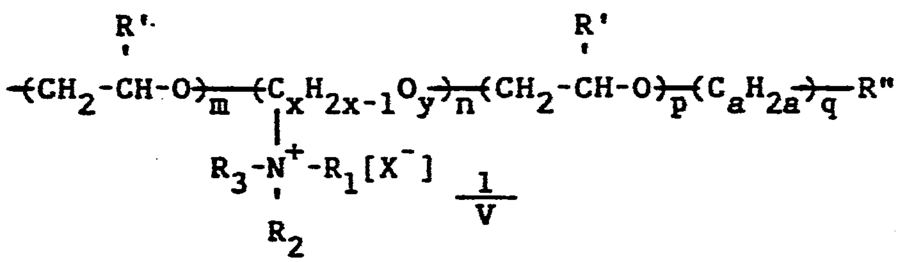

wherein, for the three R groups on the average:

the nitrogen atom is attached to a carbon atom not attached to another hetero atom;

a has a value of from 1 to 3;

m has a value of from zero to 6;

n has an average value per anhydroglucose unit of from zero to 3 and has a value other than zero and adequate to impart alcohol solubility when the average value of m+p+q is inadequate to provide alcohol solubility;

p has a value of from zero to 6;

q has a value of from zero to 3;

the average value of m+p+q per anhydroglucose unit

provides said polysaccharide with alcohol solubility when $\underline{n}$ is zero;

R' is $CH_3$ or H;

R" is a member selected from the group consisting of:

$$-H,\ -\overset{O}{\overset{\|}{C}}-OH,\ -\overset{O}{\overset{\|}{C}}-O-Na,\ -\overset{O}{\overset{\|}{C}}-O-K, \text{ and } -\overset{O}{\overset{\|}{C}}-O-NH_4$$

with the proviso that when $\underline{q}$ is zero then R" is -H;

$R_1$, $R_2$ and $R_3$ taken individually, represent a member selected from the group consisting of alkyl, aryl, aralkyl, cycloalkyl, alkoxyalkyl and alkoxyaryl radicals where each of $R_1$, $R_2$ and $R_3$ can contain up to 10 carbon atoms, with the proviso that when said member is an alkoxyalkyl radical there are at least 2 carbon atoms separating the oxygen atom from the nitrogen atom, and with the further proviso that the total number of carbon atoms in radicals represented by $R_1$, $R_2$ and $R_3$ is from 3 to 12, with the further proviso that when $R_1$, $R_2$ and $R_3$ are taken together the nitrogen atom to which $R_1$, $R_2$ and $R_3$ are attached can be a component of a heterocyclic ring selected from the group consisting of pyridine, α-methylpyridine, 2,5-dimethylpyridine, 2,4,6-trimethylpyridine, N-methylpyridine, N-ethylpyridine, N-methyl morpholine and N-ethyl morpholine;

X is an anion;

V is an integer equal to the valence of X;

$\underline{x}$ has a value from 2 to 4;

$\underline{y}$ has a value of zero to 1, with the proviso that when $\underline{y}$ is zero, $\underline{p}$ and $\underline{q}$ are zero and R" is H; and

with the proviso that when R' is $CH_3$, n has a value other than zero.

2. The polysaccharide of claim 1 wherein q is zero.

3. The polysaccharide of one or both claims 1 - 2 wherein at least two of $R_1$, $R_2$ and $R_3$ are methyl, especially each of $R_1$, $R_2$ and $R_3$ are methyl.

4. The polysaccharide of one or more of the claims 1 - 3 wherein the average value of m+p+q is at least about 3, especially in the range of from about 3.0 to about 5.5 and preferably from about 3.6 to about 4.5.

5. The polysaccharide of one or more of the claims claims 1 - 4 wherein X is chloride.

6. The polysaccharide of one or more of the claims 1 - 5 wherein n is a value providing said polysaccaride with a CS of from about 0.05 to about 0.6.

7. The polysaccharide of one or more of the claims 1 - 6 wherein z in the structural formula is selected to provide a 2 percent by weight solution in 95/5% by volume ethanol/water of said polysaccaride with a Brookfield viscosity (25°C) of less than about 6000 centipoise, especially less than about 200 centipoise, preferably less than about 160 centipoise.

8. The polysaccharide of claim 1 wherein q is zero; $R_1$, $R_2$ and $R_3$ are each methyl, X is chloride; the average value of m+p is from about 3 to about 5.5; and z in the structural formula is selected to provide a 2 percent by weight solution of said polysaccharide

in 95/5% by volume ethanol/water with a Brookfield viscosity (25°C) of less than about 6000 centipoise, especially less than about 200 centipoise.

9. A polysaccharide of one or more of the claims 1 - 8 wherein the group R'' of the general formula is -H.

10. The polysaccharide of claim 9 wherein n has a value other than 0.

11. The polysaccharide of claim 9 wherein n has a value providing said polysaccharide with a CS of from about 0.05 to about 0.6, especially with a CS of from about 0.1 to about 0.4.

12. The polysaccharide of claim 11 wherein the average value of m+p+q is from about 3.0 to about 5.5 especially from about 3.6 to about 4.5.

13. The polysaccharide of claim 1 and/or 11 wherein R' is H.

14. The polysaccharide of claim 13 wherein n has a value other than 0.

15. The polysaccharide of claim 14 wherein n has a value providing said polysaccharide with a CS of from about 0.05 to about 0.6, especially from about 0.1 to about o.4.

16. The polysaccharide of claim 13 wherein the average value of m+p+q is from about 3.0 to about 5.5, especially from about 3.6 to about 4.5.

17. A polysaccharide of claim 1 wherein in the general formula n has an average value per anhydroglucose unit of other than zero and up to 3 the average

value of m+p+q + n per anhydroglucose unit providing said polysaccharide with alcohol solubility.

18. The polysaccharide of claim 17 wherein R' is H and n has a value providing said polysaccharide with a CS of from about 0.05 to about 0.6, especially from about 0.1 to about 0.4.

19. The polysaccharide of one or both of the claims 17-18 wherein the average value of m+p+q is from about 3.0 to about 5.5, especially from about 3.6 to about 4.5.

20. In a personal care product comprising a solvent containing at least about 50% by weight alcohol and at least one active ingredient, the improvement comprising a polysaccharide according to one or more of the claims 1-19 dissolved in said solvent and present in an amount sufficient to impart conditioning characteristics to said product.

21. A hair spray formulation comprising an alcohol solvent and a polysaccharide according to one or more of the claims 1-19 dissolved in said solvent and present in an amount effective to provide hair spray characteristics.

22. The hair spray formulation of claim 21 which includes at least one propellant present in an amount sufficient to spray said polysaccharide dissolved in said alcohol solvent.

23. The hair spray formulation of one or both of the claims 21-22 wherein said polysaccharide is present in an amount of at least 0.5 percent by weight of said formulation.

24. The hair spray formualation of one or more of the claims 21-23 wherein said alcohol solvent is ethanol containing less than about 10 percent especially no greater than about 7 percent by weight water based upon the total weight of the solvent.

25. The hair spray formulation of one or more of the claims 21-24 wherein said propellant is isobutane.